# EUROPEAN PATENT SPECIFICATION

(11) **EP 0 452 123 B1**
(45) Date of publication and mention of the grant of the patent: **10.01.1996**
(21) Application number: 91303196.9
(22) Date of filing: 11.04.1991
(51) Int. Cl.: A61L 29/00

(54) **Radiopaque, optically transparent medical tubing**
Strahlungsundurchlässiges, optisch transparentes, medizinisches Rohr
Tubes médicaux radio-opaques et optiquement transparents

(30) Priority: 11.04.1990 US 508621
(43) Date of publication of application: 16.10.1991
(73) Proprietor: Becton Dickinson and Company, Franklin Lakes, New Jersey 07417-1880 (US)
(72) Inventor: Karakelle, Mutlu, Spring Valley Ohio 45370 (US); Spielvogel, David E., Springboro, Ohio 45066 (US); Lee, Min-Shiu, Spring Valley, Ohio 45370 (US); Taller, Robert A., Centerville, Ohio 45459 (US)
(74) Representative: Ruffles, Graham Keith

(56) References cited:
- EP-A- 0 233 376
- EP-A- 0 404 516
- EP-A- 0 404 517
- WO-A-82/00413
- WO-A-87/07155
- US-A- 4 282 876
- US-A- 4 657 024

## Description

1. Field of the Invention. This invention relates to medical tubing, and more particularly relates to a catheter which is transparent to light but opaque to x-rays.

2. Background. Medical tubing, principally catheters, are extensively used for administration of fluids to the body or for removal of fluids from the body. In many cases, indwelling catheters, after insertion, remain in place for protracted periods during which time they are continuously exposed to body fluids. A first prerequisite for a catheter material thus is nontoxicity, i.e., it is essential that the catheter be fabricated from a material which will not leach any toxic material into the body fluid with which it comes into contact.

It is also highly desirable that tubings to be used for body insertion be both optically transparent and radiopaque. Often, for example, the exact position of a catheter must be determined after emplacement. A convenient method for locating an emplaced catheter is by x-ray if the catheter material is radiopaque. Optical transparency is a desirable feature of catheters in order that fluids being administered to a patient or withdrawn from a patient be readily visible.

Various compositions designed to render a catheter both radiopaque and optically transparent have been disclosed in the art. Coneys, in U.S. Patent No. 4,657,024, summarizes early work on radiopaque catheters and discloses an improved catheter having a blend of an inorganic radiopaque material and a polymeric binder sandwiched between two layers of an optically transparent plastic. In an alternative embodiment of the Coneys catheter, stripes of the radiopaque blend are embedded in the plastic. The Coneys et al. catheter provides only partial optical transparency.

Goosens, in U.S. Patent No. 4,182,787, achieves radiopacity with a polycarbonate-halogenated polycarbonate copolymer.

Flynn, in U.S. Patent No. 4,579,879, discloses a radiopaque resin composition for fabrication of medical tubing which includes a polyiodobenzoic acid ester containing polyvinyl resins and a platinum-cured silicone polymer dispersed therein.

In U.S. Patent No. 4,722,344, Cambron et al. discloses a transparent radiopaque catheter made of a polyurethane containing covalently bonded high atomic number halogen atoms as part of the polymer structure.

PCT WO-A-82/00413 describes a coextruded tubing composition comprises a physiologically inert flexible water-proof thermoplastic material having encapsulated within the wall of tubing one or more strips of radiopaque material coaxially disposed along the length of tubing. The radiopaque material is bismuth trioxide, barium sulphate, or bismuth subcarbonate. Similar radiopaque materials are employed in thecatheters of US 4,657,024.

EP-A-233,376 describes iodobenzoate derivatives of use in radiopaque compositions. PCT WO-87107155 discloses catheters formed from halogenated polyurethanes.

EP-A-404,517, published 27 December 1990 describes expandable catheters made of thermoplastic elastomeric hydrophilic polyurethane. A radiopaque agent which may be an iodinated or brominated polyurethane can be included.

Significant improvements in catheter design have resulted from the compositions described in the above-referenced patents. There remains, however, a need for further improvement, particularly with respect to a catheter which is radiopaque, optically transparent and has no potentially leachable toxic materials in contact with body fluids and/or body administered fluids. It is toward fulfilment of this need that this invention is directed.

### SUMMARY OF THE INVENTION

An optically transparent and radiopaque tubing has a layer of a halogenated radiopaque material encapsulated by a non-toxic and biocompatible base polymer. In the preferred tubing, both layers are thermoplastic. The radiopaque material is a halogenated polyurethane wherein radiopacity is conferred on the tubing by halogen atoms covalently bonded to the polymer chain.

In one embodiment of the invention, one or more longitudinal stripes of the radiopaque polymer is encapsulated by the base polymer layer. In a second embodiment, the catheter of the invention has a layer of the radiopaque polymer encapsulated by layers of the base polymer laminated onto both the lumen and outside walls of the catheter.

Thus, the catheter of the invention is optically transparent to provide visibility of solutions being administered to or withdrawn from a patient and radiopaque to provide x-ray visibility of the catheter itself. Radiopacity is provided by a layer of halogenated polyurethane which is encapsulated by the polyurethane base layer so that the halogenated polymer is completely isolated from contact with air during storage or contact with a patient's blood stream during use. Release of free bromine or iodine or toxic organic compounds as a result of decomposition of the radiopaque material is prevented. Accordingly, no toxic materials can leach from the catheter into a patient's blood stream during use.

### BRIEF DESCRIPTION OF THE DRAWINGS

Fig. 1 is a perspective view of the catheter of the invention;
Fig. 2 is sectional view of the catheter of Fig. 1 taken along the line 2-2 thereof; and
Fig. 3 is a sectional view of an alternative embodiment of the catheter of Fig. 1 taken along the line 2-2 thereof.

### DETAILED DESCRIPTION

While this invention is satisfied by embodiments in many different forms, there will herein be described in detail preferred embodiments of the invention, with the understanding that the present disclosure is to be considered as exemplary of the principles of the invention and is not intended to limit the invention to the embodiments illustrated and described.

The catheter configurations of the invention which include a nontoxic biocompatible polymer in contact with a patient's blood stream and a halogenated radiopaque polymer or composition which is separated from the patient's blood stream will first be described with the aid of the figures. Subsequently, the materials used to form each of the elements will be described.

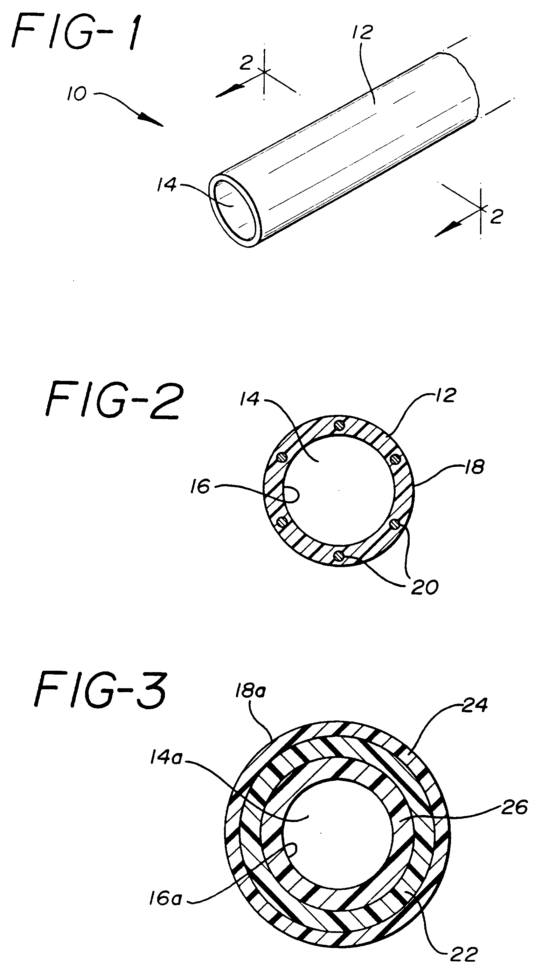

Adverting now to the drawings, Figs. 1 and 2 show a typical catheter tubing 10 having a plastic body portion 12 defining a lumen 14. Body portion 12 has a lumen wall 16 and an outside wall 18. As seen more clearly in Fig. 2, one or more stripes 20 of a radiopaque polymer is disposed longitudinally along at least a portion of the catheter length and encapsulated in body portion 12. While Fig. 2 shows the catheter to have the preferred six stripes, any convenient number of stripes from 1 to about 9 may be included.

Fig. 3 shows an alternative embodiment of the tubing of the invention in which a layer of the radiopaque polymer 22 is encapsulated by laminated layers 24 and 26 of the plastic body portion which defines lumen 14a, lumen wall 16a and outside wall 18a.

The body portion of the catheter of the invention may be any thermoplastic base polymer which is biocompatible and does not release any toxic products when in contact for prolonged periods with a body fluid. Preferred base polymers are flexible but of sufficient mechanical strength to be advanced through a tortuous body fluid path, such as a patient's blood stream. While the invention contemplates use of any polymeric or copolymeric base material which provides these attributes, preferred base materials are polyolefins, such as polyethylene, polypropylene and fluorinated ethylene propylene copolymer (FEP), polyesters, polyurethaneureas and, most preferably, polyurethanes.

The polyurethane base polymer may be a polyester polyurethane, a silicone-urethane block copolymer or preferably a polyetherurethane and is the product from reaction of a diisocyanate, a polyglycol and a chain extender.

Suitable diisocyanates are aromatic diisocyanates such as 4-4′-diphenylmethane diisocyanate, (MDI), alicyclic diisocyanates such as isophorone diisocyanate and 4-4′-dicyclohexylmethane diisocyanate, and aliphatic diisocyanates, as, for example, hexamethylene diisocyanate (HMDI).

The polyglycol component may be a polyester glycol, a silicone glycol or preferably a polyether glycol. Suitable polyester glycols are, for example, polycaprolactone and polyethyleneadipate. Suitable silicone glycols are, for example, polydimethylsiloxane-polyethylene oxide copolymers, such as Q4 3667, available from Dow Corning Co.

The preferred polyether glycol may be polyethylene oxide glycol, polypropylene oxide glycol or, most preferably, polytetramethylene oxide glycol, PTMEG. The PTMEG may have a molecular weight of about 600 to 3,300 preferably about 1,000 to 2,000. These products are available commercially under the trade names Polymeg® (Quaker Oats Co.) and Terathane® (DuPont).

The chain extender may be water and/or a low molecular weight branched or unbranched diol, diamine or aminoalcohol of up to 10 carbon atoms or mixtures thereof. Representative nonlimiting examples of chain extenders are 1,4-butanediol (BDO); ethylene glycol; diethylene glycol; triethylene glycol; 1,2-propanediol; 1,3-propanediol; 1,6-hexanediol; 1,4-bis-hydroxymethyl cyclohexane, hydroquinone dihydroxyethyl ether, ethanolamine, ethylenediamine, hexamethylenediamine and 2-methyl-pentamethylene diamine. Preferred chain extenders are 1,6-hexanediol, ethylenediamine, hexamethylenediamine and, most preferably, BDO.

Suitable polyurethane base polymers have a hard segment (HS) content of about 20% to 90%, preferably about 25% to 80%, by weight, a Shore hardness range of from about 40A to 90D preferably about 70A to 75D, and a tensile strength of about 3,000 to 10,000 psi. Calculation of component ratios from a preselected hard segment percentage to give polyurethanes within the above Shore hardness range is easily within the purview of one skilled in the art.

The radiopaque polymers are brominated or iodinated polyurethanes having a hard segment content of about 50 to 80 weight percent which may be prepared by either of the processes described below using a bromide or iodide substituted isocyanate, polyglycol or extender. Suitable halogenated isocyanates are dibromo diphenylmethane diisocyanate, tetrabromo diphenylmethane diisocyanate, dibromo dicyclohexylmethane diisocyanate and tetrabromo dicyclohexylmethane diisocyanate. Suitable halogenated extenders are brominated and iodinated aliphatic and aromatic glycols. Such halogenated glycols may of course be part of a polyether or polyester polyol. Examples of suitable halogenated starting materials and sources thereof are given in the aforementioned U.S. Patent No. 4,722,344.

The catheter of the invention may have about 30 to 60 volume percent of the halogenated polymer or composition in the form of the stripes or layer as described above.

The base polyurethane or the halogenated polyurethane of the invention may be prepared by the conventional two step or prepolymer method. As an example of this procedure, the hydroxyl containing components, i.e., the extender and polyglycol may be reacted in a suitable solvent as, for example, dimethylformamide or preferably dimethylacetamide (DMAC) with approximately two equivalents of diisocyanate so that each hydroxyl group is reacted with a diisocyanate molecule giving a prepolymer having isocyanate terminal groups (a process conventionally referred to as capping). The prepolymer molecules may then be further chain extended by reaction between their terminal isocyanate groups and the chain extender. An example of preparation of the base polymer by the prepolymer procedure is given in Example I, however, various modifications of this conventional procedure are well-known to those skilled in the art.

Alternatively, the base polymer may be prepared by a bulk or one-shot polymerization procedure. In a bulk polymerization process, all of the ingredients are combined at the beginning of the process and subjected, usually with stirring, to a polymerization initiator such as heat, catalyst, irradiation and the like.

In one suitable bulk polymerization process of the invention, conventional polymerization equipment is charged with a mixture of the polyglycol and extenders in proportions predetermined in accordance with the desired hard segment-soft segment ratio. With vigorous stirring, the diisocyanate may be added all at once. If the reaction does not start spontaneously, the mixture may be heated sufficiently to induce an exothermic reaction. The reaction mixture may be stirred vigorously until the exotherm is complete and the temperature begins to drop off, generally for about 1 to 5 minutes. The clear homogeneous melt, while still hot, may advantageously be removed from the reactor prior to curing.

In an alternative procedure, the polyglycol and diisocyanate may be mixed with stirring, and, when the initial exotherm begins to subside, the extender may be added with continued stirring.

The catheter of the invention may be fabricated from the base polymer and the clear radiopaque component by any convenient coextrusion process. A base polymer melt stream from a main extruder and a clear radiopaque polymer melt stream from a coextruder may be maintained separately until combined as continuous stripes or layers in the forward, down stream portion of an extruder head. From the extruder head, the streams subsequently pass through and emerge from a tube die (coaxial or cross-head) as an integral tubing member.

By proper selection of extruders, coextruders and dies, the number and thickness of the stripes, layers or other configurations and thereby the volume percentages of the base polymer and the clear radiopaque component may be adjusted according to the requirements of the particular coextruded tubing.

The following examples are given to further describe the invention but are not to be considered as limitative of the invention.

### EXAMPLE I

### General Procedure for Base Polymer Synthesis

The calculated quantities of polyglycol and BDO required to prepare a base polyurethane having the desired hard segment content were combined in a resin bottle at 60°C, vacuum stripped for 30 minutes at 4 mm Hg. The mixture was cooled to ambient temperature and stoichiometric equivalents of MDI, based on the total hydroxyl content, was added all at once with vigorous stirring. The exotherm reached about 80°C, whereupon the mixture was poured into a Teflon-lined tray and post-cured at 110°C for about 60 minutes.

In an alternative procedure, the polyglycol and diisocyanate were mixed with stirring, and, when the initial exotherm began to subside, the extender was added with continued stirring.

### EXAMPLE II

### General Procedure for Halogenated Polyurethane Synthesis

The calculated quantities of polyglycol and brominated neopentyl glycol required to prepare a polyurethane having the desired hard segment content were combined in a resin bottle at 60°C, vacuum stripped for 30 minutes at 4 mm Hg. The mixture was cooled to ambient temperature and stoichiometric equivalents of MDI, based on the total hydroxyl content, was added all at once with vigorous stirring. The exotherm reached about 80°C, whereupon the mixture was poured into a Teflon-lined tray and post-cured at 110°C for about 60 minutes.

In an alternative procedure, the polyglycol and diisocyanate were mixed with stirring, and, when the initial exotherm began to subside, the brominated neopentyl glycol was added with continued stirring.

### EXAMPLE III

### General Procedure for Preparation of Clear Radiopaque Compositions

Clear radiopaque polymeric compositions were compounded in 7 lb. batches using a Banbury mixer. The desired matrix polymer and halogenated organic compound were weighed, dry-mixed and put into the mixing chamber where they were compounded at 60 RPM. The mixing chamber temperature was maintained between 150 and 190°F. The mixture was allowed to flux for 3 to 5 minutes, and then the rotor speed was reduced to 20 RPM and the jacketed mixing chamber was chilled with water. At approximately 130°F, the mixture was taken out of the mixing chamber and allowed to cool to room temperature. The composition was immersed in liquid nitrogen and then granulated on a Foremost Model SHD-1 grinder. The chipped composition was dried prior to the coextrusion runs.

For liquid halogenated organic radiopaque compounds, an alternative method was used. The matrix polymer was mixed with the liquid and maintained at elevated temperatures (100 to 200°C) to facilitate the absorption of the liquid compound into the resin, usually about 12 to 24 hours.

### EXAMPLE IV

### Radiopacity of 20 Gauge Optically Transparent Medical Tubing

Twenty gauge six stripe medical tubings having 35% by volume of the radiopaque material were prepared by coextrusion of a nontoxic biocompatible thermoplastic polyurethane base polymer and clear radiopaque polymeric compounds as the stripe material. The opacity to x-ray of these tubings was determined with respect a brass rod standard. The tubing samples were exposed to x-ray at 70 kilovolts (at the peak) and 10 milliampere seconds. The x-ray films were developed and images were scanned three times using an optical scanning densitometer. Average peak heights of the samples were compared to that of the brass standard rod.

**TABLE I**

| 20 Gauge Sample Tubing Base Polymer/Clear Radiopaque Stripe | | % Radiopacity of Brass Standard |
|---|---|---|
| 1) | Base Polyurethane* (Negative Control) | 0 |
| 2) | Brass Standard (Cu/Zn:70/30; 0.079" wide 0.003" thick) | 100.0 |
| 3) | Base Polyurethane/Brominated Polyurethane (74% HS, HMDI, Bromoneopentyl Glycol, PTMEG-2000 | 10.7 |
| 4) | Base Polyurethane/Polyurethane-Diiodobenzoic Acid Ester (70:30 by weight) | 26.4 |
| * (74% HS, HMDI, PTMEG-2000, BDO) | | |

### EXAMPLE V

The polyurethane catheter of the invention and a prior art catheter fabricated from only brominated radiopaque polyurethane were set aside for 6 months with exposure to the atmosphere at ambient conditions, i.e., conditions which simulate shelf life. The prior art catheter developed a strong smell of bromine. The catheter of the invention was devoid of any halogen smell for the 6 months and longer.

### EXAMPLE VI

### Cytotoxicity Studies

In a study conducted at North American Science Asociation (Northwood, Ohio), 20 gauge 6 stripe medical tubings of the invention (entries 3 and 4 of Example IV) were compared with two prior art tubings in which the halogenated radiopaque material is not separated from the environment.

Procedure: A monolayer of L-929 mouse fibroblast cells was grown to confluency and exposed to an extract of the test article prepared by placing the test article in 20 ml of Minimum Essential Medium (MEM) (Eagle) and bovine serum (5%) and extracting at 37°C for 24 hours. An MEM aliquot was used as a negative control. After exposure to the extract, the cells were examined microscopically for cytotoxic effect. Presence or absence of a confluent monolayer, intracellular granulation, cellular swelling and crenation and the percentage of cellular lysis were recorded at 24,48 and 72 hours. Tubing extracts causing greater than 50% cell lysis were considered to be toxic. The results of this study are given in Table II.

**TABLE II**

| | SAMPLE | CELL LYSIS % | | | CYTOTOXICITY |
|---|---|---|---|---|---|
| | | 24hr | 48hr | 72hr | |
| 1) | Base Polyurethane/Brominated Polyurethane (74% HS, HMDI, Bromoneopentyl Glycol, T-2000) Tubing | 0 | 0 | 0 | nontoxic |
| 2) | Base Polyurethane/Polyurethane-Diiodobenzoic Acid Ester Compound (70:30 by weight) Tubing | 0 | 0 | 0 | nontoxic |
| 3) | Brominated Polyurethane (74% HS, HMDI, Bromoneopentyl Glycol, T-2000) Rod | 0 | 25 | 100 | toxic |
| 4) | Polyurethane-Diiodobenzoic Acid Ester Compound (70:30 by weight) Rod | 0 | 60 | | toxic |
| * (74% HS, HMDI, PTMEG-2000, BDO) | | | | | |

From Table II, it is seen that tubings 1 and 2 of the invention where the halogen is completely isolated from the extracting medium were nontoxic whereas tubings 3 and 4 of the prior art were toxic.

## Claims

1. A catheter comprising a radiopaque halogenated polyurethane with a hard segment content of 50 to 80 weight per cent, said halogenated polyurethane being encapsulated by a layer of optically transparent polyurethane and comprising a brominated or iodinated diisocyanate, polyglycol or extender, with the proviso that the optically transparent polyurethane does not comprise the reaction product of a diisocyanate, a polyglycol component comprising at least 50% polyethylene glycol and a chain extender.

2. The catheter of claim 1 wherein said halogenated diisocyanate is selected from the group consisting of dibromo diphenylmethane diisocyanate, tetrabromo diphenylmethane diisocyanate, dibromo dicyclohexylmethane diisocyanate and tetrabromo dicyclohexylmethane diisocyanate.

3. The catheter of claim 1 wherein said halogenated polygycol is a polyether or polyester glycol.

4. The catheter of claim 1 wherein said halogenated extender is a brominated or iodinated aliphatic or aromatic glycol.

5. The catheter of claim 4 wherein said brominated aliphatic glycol is dibromoneopentyl glycol.

6. The catheter of any preceding claim, wherein the encapsulated radiopaque halogenated polyurethane is present as one or more longitudinal stripes encapsulated by the layer of optically transparent polyurethane.

## Patentansprüche

1. Katheter, der folgende Komponenten aufweist: ein strahlungsundurchlässiges, halogeniertes Polyurethan mit einem Hartsegmentgehalt von 50 bis 80 Gew.-%, wobei das halogenierte Polyurethan durch eine Schicht eines optisch transparenten Polyurethans eingekapselt ist und ein bromiertes oder iodiertes Diisocyanat, Polyglykol oder Streckmittel umfaßt, unter der Bedingung, daß das optisch transparente Polyurethan nicht das Reaktionsprodukt eines Diisocyanats aufweist, eine Polyglykolkomponente, die wenigstens 50 % Polyethylenglykol aufweist, und einen Kettenverlängerer.

2. Katheter nach Anspruch 1, bei dem das halogenierte Diisocyanat aus der Gruppe ausgewählt wird, die aus Dibromdiphenylmethan-Diisocyanat, Tetrabromdiphenylmethan-Diisocyanat, Dibromdicyclohexylmethan-Diisocyanat und Tetrabromdicyclohexylmethan-Diisocyanat besteht.

3. Katheter nach Anspruch 1, bei dem das halogenierte Polyglykol ein Polyether- oder Polyesterglykol ist.

4. Katheter nach Anspruch 1, bei dem das halogenierte Streckmittel ein bromiertes oder iodiertes aliphatisches oder aromatisches Glykol ist.

5. Katheter nach Anspruch 4, bei dem das bromierte aliphatische Glykol Dibromneopentyl-Glykol ist.

6. Katheter nach einem der vorhergehenden Ansprüche, bei dem das eingekapselte, strahlungsundurchlässige halogenierte Polyurethan in Form von einem oder mehreren Längsstreifen, die durch die Schicht des optisch transparenten Polyurethans eingekapselt werden, vorhanden ist.

## Revendications

1. Cathéter comprenant un polyuréthane halogéné radio-opaque avec une teneur en segments durs de 50 à 80 pour cent en poids, ledit polyuréthane halogéné étant encapsulé par une couche de polyuréthane optiquement transparent et comprenant un diisocyanate bromé ou iodé, un polyglycol ou un agent d'extension, à condition que le polyuréthane optiquement transparent ne comprenne pas le produit de réaction d'un diisocyanate, un composant de polyglycol comprenant au moins 50% de polyéthylène-glycol et un agent d'extension de chaîne.

2. Cathéter selon la revendication 1, dans lequel ledit diisocyanate halogéné est choisi dans le groupe constitué du diisocyanate de dibromo-diphénylméthane, du diisocyanate de tétrabromo-diphénylméthane, du diisocyanate de dibromo-dicyclohexylméthane et du diisocyanate de tétrabromo-dicylcohexylméthane.

3. Cathéter selon la revendication 1, dans lequel ledit polyglycol halogéné est un polyéther-glycol ou un polyester-glycol.

4. Cathéter selon la revendication 1, dans lequel ledit agent d'extension halogéné est un glycol bromé ou iodé aliphatique ou aromatique.

5. Cathéter selon la revendication 4, dans lequel ledit glycol aliphatique bromé est le dibromonéopentyl-glycol.

6. Cathéter selon l'une quelconque des revendications précédentes, dans lequel le polyuréthane halogéné radio-opaque encapsulé est présent sous forme d'une ou de plusieurs bandes longitudinales encapsulées par la couche de polyuréthane optiquement transparent.
